# EUROPEAN PATENT APPLICATION

(11) **EP 1 221 325 A2**
(43) Date of publication of application: **10.07.2002**
(21) Application number: 02250129.0
(22) Date of filing: 09.01.2002
(51) Int. Cl.: A61P 17/02, A61K 35/78

(54) **A wound healing antiscarring topical composition of centella and ginseng**

(30) Priority: 09.01.2001 IN BO002301
(71) Applicant: Johnson & Johnson Limited, Mumbai 400036, Maharashtra (IN)
(72) Inventor: Alain, Khaiat, c/o Johnson & Johnson Asia Pacific, 29-01 Suntec Tower 3, Singapore 038988 (SG); Sitaram, Manke Ajit, c/o Johnson & Johnson Limited, Mumbai 400080, Maharashtra (IN); Mini, Thomas , c/o Johnson & Johnson Limited, Mumbai 400080, Maharashtra (IN); Shankar, Telang Ajit, c/o Johnson & Johnson Ltd., Mumbai 400080, Maharashtra (IN); Nayaran, Abhyankar Prashant,, Mumbai 400080, Maharashtra (IN)
(74) Representative: Mercer, Christopher Paul

(57) **Abstract**

A wound healing antiscarring topical composition of centella and ginseng. It comprises centella whole extract in 0.01 - 1% by weight and ginseng extract in 0.01 - 0.5% by weight as actives and optionally formulating agents. A process for the preparation of the composition which comprises mixing the actives centella whole extract in 0.01 - 1% by weight and ginseng extract in 0.01 - 0.5% by weight optionally with formulating agents at 25-70°C. A wound healing antiscarring medicated pad of centella and ginseng. The pad comprises a permeable material strip impregnated with centella whole extract in 0.01 - 1 % weight/weight and ginseng extract in 0.01 - 0.5% weight/weight as actives. A method of making the pad which comprises dissolving the actives centella whole extract and ginseng extract in a solvent such that the solution contains 0.008 - 0.8% and 0.008 - 0.4% weight/weight of the actives. A permeable material strip is impregnated with the solution of the actives such that the percentage weight pick-up or add-on weight thereon is 130 ± 40%. The medicated strip is dried to evaporate the solvent.

## Description

### FIELD OF INVENTION

This invention relates to a wound healing antiscarring topical composition of centella and ginseng.

This invention also relates to a process for the preparation of the topical composition, a wound healing antiscarring medicated pad of centella and ginseng and a method of making the medicated pad.

The topical composition/medicated pad of the invention is for therapeutic use on wound, surgical cut, ulcer or the like.

### PRIOR ART

The herb centella asiatica is well known to have medicinal properties. Its extract viz centella extract is reported to be used in preventing and healing of various types of wounds or ulcers, disinfection thereof, in cicatrisation by regeneration or stimulation of skin and also as a scar minimising agent ("Clinical study of a new antikeloid agent", Annals of Plastic Surgery, Vol 3, No 1, pp 13 to 21, by Bosse J P etal, French Patent No 2594690A, European Patent No 22046A, Belgium Patent No 699410A). For therapeutic use, usually the centella extract isolated from the herb is purified further by nanofiltration, liquid-liquid extraction or column chromatography to obtain an extract enriched with its essential triterpene actives viz asiaticoside, asiatic acid and madecassic acid. Such purified centella extract comprising 1% of the triterpenes is known to be formulated to cream or gel with formulating agents such as lanoline or propylene glycol oleostearate. Alternatively at the time of use, such cream or gel of purified centella extract may be manually applied onto a permeable material strip and affixed as bandage to the affected area of skin. Purification of centella extract is laborious, time consuming and expensive thus rendering purified centella extract very expensive. Although purified centella extract is reported to show efficacy at ∼ 1% of the triterpenes, the cost of the purified extract and that too at 1% is high and further increases the cost of the formulations.

The extract obtained from the roots of the ginseng plant is reported to be mainly effective in scar elimination/reduction, prevention of roughening of skin and is also reported to have use in curing ulcer (Chinese Patents Nos 1105267A, 1104536A and 1080181, European Patent No 685229, Japanese Patents Nos 6239759A, 5262635A and 58044058A and US Patent No 5658578A). The activity of the ginseng extract is attributed to the saponins contained therein. Ginseng extract in 0.005 - 10 % is reported to show efficacy.

### OBJECTS OF INVENTION

An object of the invention is to provide a wound healing antiscarring topical composition of centella and ginseng, which shows enhanced wound healing with reduced scar formation and that too at low concentrations of the actives.

Another object of the invention is to provide a wound healing antiscarring topical composition of centella and ginseng, which is comparatively less expensive.

Another object of the invention is to provide a process for the preparation of a wound healing antiscarring topical composition of centella and ginseng which shows enhanced wound healing with reduced scar formation and that too at low concentrations of the actives.

Another object of the invention is to provide a process for the preparation of a wound healing antiscarring topical composition of centella and ginseng which is comparatively less expensive.

Another object of the invention is to provide a wound healing antiscarring medicated pad of centella and ginseng, which shows enhanced wound healing with reduced scar formation and that too at low concentrations of the actives.

Another object of the invention is to provide a wound healing antiscarring medicated pad of centella and ginseng, which is comparatively less expensive.

Another object of the invention is to provide a method of making a wound healing antiscarring medicated pad of centella and ginseng which shows enhanced wound healing with reduced scar formation and that too at low concentrations of the actives.

Another object of the invention is to provide a method of making a wound healing antiscarring medicated pad of centella and ginseng which is comparatively less expensive.

According to the invention, there is provided a wound healing antiscarring topical composition of centella and ginseng comprising centella whole extract in 0.01 - 1% by weight and ginseng extract in 0.01 - 0.5% by weight as actives optionally in conjunction with formulating agents.

According to the invention there is also provided a process for the preparation of a wound healing antiscarring topical composition of centella and ginseng comprising mixing the actives centella whole extract in 0.01 - 1% by weight and ginseng extract in 0.01 - 0.5% by weight optionally with formulating agents at 25-70°C.

According to the invention there is also provided a wound healing antiscarring medicated pad of centella and ginseng comprising a permeable material strip impregnated with centella whole extract in 0.01 - 1% weight/weight and ginseng extract in 0.01 - 0.5% weight/weight as actives.

According to the invention there is also provided a method of making a wound healing antiscarring medicated pad of centella and ginseng comprising a permeable material strip impregnated with centella whole extract in 0.01 - 1% weight/weight and ginseng extract in 0.01 - 0.5% weight/weight as actives, the method comprising:
a) dissolving the actives centella whole extract and ginseng extract in a solvent such that the solution contains 0.008 - 0.8% and 0.008 - 0.4% weight/weight of the actives respectively;
b) impregnating the permeable material strip with the solution of the actives such that the percentage weight pick-up or add-on weight thereon is 130 ± 40 %; and
c) drying the medicated strip to evaporate the solvent.

The term whole extract of centella means crude extract isolated from plant in known manner or commercially available and unpurified. The whole extract of centella and ginseng extract may be water or glycolic extracts spray dried or otherwise.

The composition of the invention may be converted to forms such as gel or cream in known manner using formulating agents such as sodium acid phosphate, propylene glycol, methyl or propyl paraben, glycerine, nitrosol, polyethylene glycol and/or polysorbate 20.

The centella whole extract in the composition may be in, preferably 0.1- 1% by weight.

The ginseng extract in the composition may be in, preferably 0.1 - 0.5% by weight.

The permeable material strip in the medicated pad comprises preferably 0.1 - 1% weight/weight of the centella whole extract and 0.5 - 1% weight/weight of the ginseng extract.

The permeable material strip may be flannelette cloth or nonwoven fabric.

Solvents used for dissolving the centella whole extract or the ginseng extract may be propanol, ethyl acetate and/or water. Preferably centella and ginseng whole extracts are dissolved in water: propanol: ethyl acetate :: 2:1:1.

Preferably the solution of the actives comprises 0.5 - 0.8% by weight of the centella whole extract and 0.3 - 0.4% by weight of the ginseng extract.

Preferably the percentage weight pick-up or add-on weight on the permeable material strip is 130%.

The medicated strip may be used as such as a bandage. Alternatively the medicated strip may be used in a typical adhesive bandage which comprises a backing layer provided with breathing perforations and coated with an adhesive at the inner surface thereof. The medicated permeable strip of the invention can be stuck to the inner surface of the backing layer and covered with a non-stick cover and peel-off strips. The method of making the typical adhesive bandage except the medicated permeable strip, may be in known manner. The medicated pad may also be of the non-occlusive type as described in our Indian Patent Application No 851/MUM/2000. The non-occlusive medicated pad may also used in a typical adhesive bandage as described above. These combinations are to be construed to be within the scope of the invention.

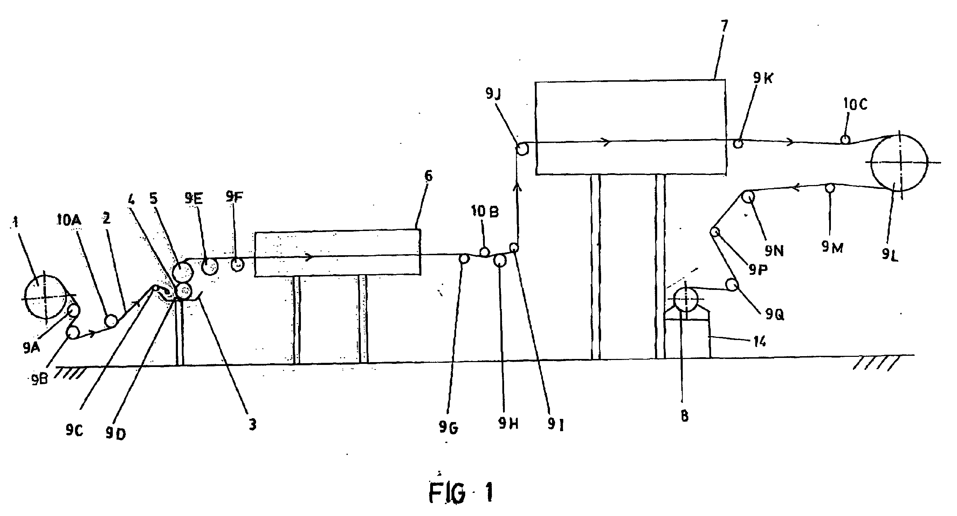

Fig 1 of the accompanying drawings is a schematic flow diagram of an impregnation system used for carrying out the method of the invention. The impregnation system is known and works in known manner.

In Fig 1, 1 is the roller for unwinding a permeable material roll 2. 3 is a trough containing a solution (not shown) of actives viz centella whole extract and ginseng extract in a mixture of water, propanol and ethylacetate. 4 and 5 are contra-rotating squeezing rollers disposed in the trough one above the other defining a nip or clearance (not shown) therebetween. 6 and 7 are drying ovens. 8 is a roller for winding the dried permeable material roll impregnated with the actives. 9A-9N, 9P and 9Q are guider rollers. 10A, 10B and 10C are tension rollers.

When the permeable material roll 2 passes through the solution comprising the actives contained in trough 3 it gets impregnated with the actives. The nip between the squeezing rollers is adjusted to control the percentage weight pick-up or add-on weight on the permeable material roll. The squeezing rollers also ensure a uniform impregnation of the actives on the permeable material roll. The impregnated and dried permeable material roll on roller 8 is cut to strips (not shown) of the required sizes.

Other known devices may be used for impregnation of the permeable strip. Such variation is within the scope of the invention.

The centella whole extract and ginseng extract combination of the invention in the weight percentages as stated herein exhibits fast wound healing with reduced scar formation. The medicated pad of the invention uniformly impregnated with such combination also exhibits fast wound healing with reduced scar formation. The efficacy of the composition of the invention is confirmed from the following invitro and invivo studies. The actives in combination have been found to show activity at as low as 0.01% concentration itself. The cost of the centella whole extract being nearly one hundredth that of the purified extract, the composition/medicated pad of the invention is rendered comparatively less expensive. Besides, the use of the whole extract of centella eliminates the purification step and saves time thereby further reducing the cost of the composition/medicated pad of the invention. The presence of other actives in centella whole extract besides the triterpenes, also may be responsible for the enhanced wound healing cum reduced scarring properties of the composition/medicated pad of the invention and that too at low concentrations.

The following experimental examples are illustrative of the invention but not limitative of the scope thereof.

### Example 1

Centella (C) whole extract [of Amsar Private Limited, India, 0.024 gm] and ginseng (G) extract [of Indena Private Limited, India, 0.024 gm] were mixed at 30°C to obtain 24 ml of topical composition comprising 0.1% by weights of each of C whole extract and G extract.

### Example 2

The following ingredients were mixed at 65°C to obtain a topical gel:

| | | |
|---|---|---|
| Sodium acid phosphate | - | 0.8 % |
| Dried sodium phosphate | - | 0.04 % |
| Glycerine | - | 11.25 % |
| Propylene glycol | - | 3.75 % |
| Propyl paraben | - | 0.04 % |
| Methyl paraben | - | 0.1 % |
| Nitrosol | - | 1.98 % |
| Sodium alginate | - | 0.001 % |
| Edetate di sodium | - | 0.015 % |
| Purified water | - | 81.824 % |
| Centella whole extract | - | 0.1 % |
| Ginseng extract | - | 0.1 % |

### Example 3

The procedure of Example 1 was followed with 0.0012 gm of each of C whole extract and G extract to obtain 2.4 ml of topical composition comprising 0.005% by weights of each of C whole extract and G extract.

### Example 4

The following ingredients were mixed at 65°C to obtain topical gel.

| | | |
|---|---|---|
| Sodium acid phosphate | - | 0.8 % |
| Dried sodium phosphate | - | 0.04 % |
| Glycerine | - | 11.25 % |
| Propylene glycol | - | 3.75 % |
| Propyl paraben | - | 0.04 % |
| Methyl paraben | - | 0.1 % |
| Nitrosol | - | 1.98 % |
| Sodium alginate | - | 0.001 % |
| Edetate di sodium | - | 0.015 % |
| Purified water | - | 80.874 % |
| Centella whole extract | - | 0.1 % |
| Ginseng extract | - | 0.05 % |

### Example 5

The procedure of Example 1 was followed with 0.012 gm of each of C whole extract and G extract to obtain 24 ml of topical composition comprising 0.05% by weights of each of C whole extract and G extract.

### Example 6

C whole extract (0.024 kg) and G extract (0.012 kg) were dissolved in a mixture of water (15 kg), propanol (7.5 kg) and ethylacetate (7.5 kg) to contain 0.08% by weights of each of C whole extract and G extract in the solution. The percentage weight pick-up or add-on weight on the permeable strip comprising flannelette cloth was 130% and the medicated flannelette cloth strip was dried in ovens progressively at 50 - 80°C. The dried medicated strip piece was found to contain 0.1% weight/weight of C whole extract and 0.05% weight/weight of G extract.

### Example 7

The procedure of Example 6 was followed by dissolving 0.012 kg of each of C whole extract and G extract in solvent. (water =15 kg, propanol = 7.5 kg, ethylacetate = 7.5 kg). The percentage weight pick up or add-on weight on the flannelette cloth strip was 130%. The medicated strip piece was found to contain 0.1% weight/weight of each of C whole extract and G extract.

### IN-VITRO STUDIES:

The following parameters were studied to test the efficacy of the compositions of the invention.

### Studies on conversion of Latent TGF - beta to active form:

Active TGF-beta (Transforming Growth Factor-beta) is an important factor in the process of scarring and its inhibition reduces scarring. Lipopolysaccharide (LPS) stimulated mouse macrophages were fibroblast conditioned. These were treated with 0.001%, 0.01% and 0.1% by weights of each of whole extracts of C (Mfd by Amsar Private Limited, India), G extracts (Mfd by Indena Private Limited, India) and purified C extracts (Mfd by Indena Private Limited, India) and 0.001% of a composition of C whole extract + G extract; composition of Example 3 comprising 0.01% by weight of C whole extract + G extract and composition of Example 5 comprising 0.1% by weight of C whole extract + G extract.

The reductions in TGF-beta levels were as follows in Table 1:

**Table 1**

| | | | |
|---|---|---|---|
| Concentration | 10 µg (0.001%) | 100 µg (0.01%) | 1000 µg (0.1%) |
| C whole extract | 47% | 81% | 86% |
| G extract | No effect | No effect | 26% |
| C whole extract + G extract | 19.5% | 49.4% | 61.7% |
| C purified extract | No effect | No effect | No effect |

From the above results it is clear that C whole extract at 0.1% and 0.01% showed marked reduction in the conversion of latent TGF-beta to the active form while G extract showed slight reduction and at 0.1%. The compositions of Examples 3 and 5 showed ∼ 50% and ∼ 62% suppression of TGF-beta activation respectively and also showed better activity when compared to the purified C extract which had no effect at all in all the concentrations stated above. It is important to note that even though 0.1% and 0.01% of C whole extracts caused nearly total suppression of TGF-beta activation, the 62% inhibition obtained using the composition of Example 5 is sufficient to inhibit scarring while allowing the low levels of TGF-beta to function in the normal process of healing. Therefore the concentrations of the actives in the compositions as per the invention are optimal to be highly effective in healing of wound and at the same time reduce scar formation.

### Studies showing the anti-inflammatory (antiscarring) activity:

Macrophages conditioned in RPMI + 10% FBS medium were utilised for inhibition of IL-6 (Interleukin - 6) levels by treating with 0.001%, 0.01% and 0.1% by weights of each of whole extracts of C ( Mfd by Amsar Private Limited); G extracts (Mfd by Indena Private Limited, India) and purified C extracts (Mfd by Indena Private Limited, India) and 0.001% of a composition of C whole extract + G extract; composition of Example 3 comprising 0.01% by weight of C whole extract + G extract and composition of Example 5 comprising 0.1% by weight of C whole extract + G extract.

The results of reduction in IL- 6 levels were as follows in Table 2.

**Table 2**

| | | | |
|---|---|---|---|
| Concentrations | 10 µg (0.001%) | 100 µg (0.01%) | 1000 µg (0.1%) |
| C whole extract | 5.6% | 22% | 27.8% |
| G extract | No effect | No effect | 27.7% |
| C whole extract + G extract | No effect | 22% | 35% |
| C purified extract | No effect | No effect | No effect |

From the above results it is very clear that the compositions of Examples 3 and 5 of the invention, specially that of Example 5 was found to be more effective in inhibiting IL-6 levels. This implies more efficacy in reducing inflammatory signals and increased rate of wound healing when compared to the individual C and G whole extracts, whereas purified C extracts showed no effect at all.

### In-vivo studies

Studies were conducted on surgical wounds of sizes 2" x 1" - 10" x 1" of 30 human volunteers. 15 volunteers were treated with a placebo base (plain gel) while 15 others were treated with the composition of the invention of Example 5. The data was tested using the "t" test at 95% confidence level.

The mean healing times of the placebo group and groups treated with the composition of Example 5 were 13.64 days and 10.64 days respectively. These results using the compositions of the invention were found to be statistically significant. 30 volunteers visually rated the degree of scarring on a scale from 1 - 5 (1 rated low scarring and 5 rated very high scarring), based on photographs taken before and after the stitches were removed after healing times. The average rating by the volunteers for the treated sets was 2.664 whereas for the placebo sets was 4.541. This showed that the degree of scarring in the treated sets was much less when compared to placebo.

The centella whole extract and ginseng extract combination as per the invention has been found to be effective in both fast wound healing and reduced scarring even at low concentrations of the actives i.e. from 0.01%.

## Claims

1. A wound healing antiscarring topical composition of centella and ginseng comprising centella whole extract in 0.01 - 1% by weight and ginseng extract in 0.01 - 0.5% by weight as actives optionally in conjunction with formulating agents.

2. A composition as claimed in claim 1, wherein the centella whole extract is in 0.1 - 1% by weight and the ginseng extract is in 0.1 - 0.5% by weight.

3. A composition as claimed in claim 1 or 2, which when combined with formulating agents is a gel.

4. A composition as claimed in claim 1 or 2, which when combined with formulating agents is a cream.

5. A process for the preparation of a wound healing antiscarring topical composition of centella and ginseng comprising mixing the actives centella whole extract in 0.01 - 1% by weight and ginseng extract in 0.01 - 0.5% by weight optionally with formulating agents at 25-70°C.

6. A process as claimed in claim 5, wherein the centella whole extract is in 0.1 - 1% by weight and the ginseng extract is in 0.1 - 0.5% by weight.

7. A process as claimed in claim 5 or 6, wherein the composition using formulating agents is a gel.

8. A process as claimed in claim 5 or 6, wherein the composition using formulating agents is a cream.

9. A process for the preparation of a wound healing antiscarring topical composition of centella and ginseng substantially as herein described particularly with reference to Examples 1 to 5.

10. A wound healing antiscarring medicated pad of centella and ginseng comprising a permeable material strip impregnated with centella whole extract in 0.01 - 1% weight/weight and ginseng extract in 0.01 - 0.5% weight/weight as actives.

11. A medicated pad as claimed in claim 10, wherein the centella whole extract is in 0.1 - 1% weight/weight and the ginseng extract is in 0.1 - 0.5% weight/weight.

12. A method of making a wound healing antiscarring medicated pad of centella and ginseng comprising a permeable material strip impregnated with centella whole extract in 0.01 - 1% weight/weight and ginseng extract in 0.01 - 0.5% weight/weight as actives, the method comprising:
a) dissolving the actives centella whole extract and ginseng extract in a solvent such that the solution contains 0.008 - 0.8% and 0.008 - 0.4% weight/weight of the actives respectively;
b) impregnating the permeable material strip with the solution of the actives such that the percentage weight pick-up or add-on weight thereon is 130 ± 40 %; and
c) drying the medicated strip to evaporate the solvent.

13. A method as claimed in claim 12, wherein the medicated permeable strip is impregnated with 0.1 - 1% weight/weight of the centella whole extract and 0.1 - 0.5% weight/weight of the ginseng extract.

14. A method as claimed in claim 12 or 13, wherein the solvent for dissolving the centella whole extract or ginseng extract is water: propanol : ethyl acetate :: 2:1:1.

15. A method as claimed in any one of claims 12 to 14, wherein the solution of the actives contains 0.5 - 0.8% weight/weight of the centella whole extract and 0.3 - 0.4% weight/weight of the ginseng extract.

16. A method as claimed in any one claims 12 to 15, wherein the percentage weight pick-up or add-on weight on the permeable material. strip is 130%.
